# FASCICULE DE BREVET EUROPEEN

(11) **EP 3 344 750 B1**
(45) Date de publication et mention de la délivrance du brevet: **16.11.2022**
(21) Numéro de dépôt: 16767003.3
(22) Date de dépôt: 31.08.2016
(51) Int. Cl.: C12N 1/20, C12M 1/00, C12M 1/26, C12M 1/34

(54) **APPAREIL PRÉSSURISÉ DONT LE COUVERCLE PRÉSENTE UN EMBOUT DE PRÉLÉVEMENT COMPRENANT AU MOINS UN ORIFICE OBTURABLE**
UNTER DRUCK STEHENDES GERÄT MIT EINER ABDECKUNG MIT EINER PROBENAHMESPITZE MIT MINDESTENS EINER VERSCHLIESSBAREN ÖFFNUNG
PRESSURISED APPLIANCE WITH A COVER COMPRISING A SAMPLING TIP HAVING AT LEAST ONE CLOSABLE OPENING

(30) Priorité: 03.09.2015 FR 1558154
(43) Date de publication de la demande: 11.07.2018
(73) Titulaire: Biomérieux, 69280 Marcy-L'Etoile (FR)
(72) Inventeur: LE RICQUE, Guilhem, 35000 Rennes (FR)
(74) Mandataire: bioMérieux PI Groupement mandataires
(86) Numéro de dépôt international: PCT/FR2016/052159
(87) Numéro de publication internationale: WO 2017/051090

(56) Documents cités:
- US-A1- 2014 377 417
- Morscher: "Mediaclave Mode d'emploi", , 9 avril 2013 (2013-04-09), XP055282845, Extrait de l'Internet: URL:http://www.integra-biosciences.com/sit es/pdf/operating_instructions/136950_V10_O I_MEDIACLAVE_FR.pdf [extrait le 2016-06-22]
- Biomerieux: "Media Preparation Masterclave 09 Media Preparator", , 1 janvier 2012 (2012-01-01), XP055282855, Extrait de l'Internet: URL:http://www.dwscientific.co.uk/assets/p roduct-pdfs/Masterclave%20Media%20Preparat ors/Masterclave%2009/010_3%20Masterclave%2 009.pdf [extrait le 2016-06-22]

## Description

La présente invention se rapporte au domaine des appareils pressurisés, notamment aux appareils de préparation de milieux de culture ou de diluants, plus particulièrement de milieux de culture en poudre. Ces appareils, également appelés auto-préparateurs de milieux de culture sont des instruments utilisés notamment par les laboratoires pour la fabrication de milieux de culture à partir de milieux déshydratés. L'invention concerne plus particulièrement un appareil pressurisé comprenant un embout de prélèvement permettant d'empêcher le débordement du contenu de l'appareil.

Le principe de fonctionnement des auto-préparateurs de milieux de culture est le suivant : l'opérateur mélange du milieu de culture déshydraté avec de l'eau dans un seau plongé dans une cuve pouvant être fermée par un couvercle. De l'eau est ajoutée dans la cuve afin de réaliser un chauffage au bain marie du contenu du seau. Un agitateur magnétique est disposé au fond du seau. Une fois le couvercle de la cuve fermé hermétiquement, l'appareil commence par mélanger l'eau et le milieu de culture déshydraté puis va stériliser le produit en le chauffant pendant une durée définie. Le contenu de la cuve atteint alors une pression environ comprise entre 1 bar et 1,5 bar. Après refroidissement, le milieu est ensuite prélevé directement dans le seau de l'auto-préparateur puis coulé à chaud dans des boites de Pétri dans le cas d'un milieu gélosé ou conservé sous forme liquide dans le cas d'un bouillon.

La qualité et la fertilité des milieux de culture préparés, qu'il soient sous forme de gélose ou de bouillon, dépend donc directement de la capacité de l'auto-préparateur à assurer une régulation précise de la température et de la pression lors de la préparation. D'autre part, la température du milieu en préparation atteignant généralement 121°C et celle-ci étant sous pression lors de cette opération, il est essentiel qu'un auto-préparateur garantisse la sécurité de l'opérateur et de son environnement de travail en empêchant tout épanchement du contenu du seau avant, pendant ou à la suite de sa préparation. D'autre part, il est essentiel qu'un auto-préparateur garantisse la stérilité du milieu préparé jusqu'à l'étape de prélèvement.

Les auto-préparateurs actuellement commercialisés par la demanderesse, tel que le Masterclave M09 (bioMérieux, Rèf: AESAP1080 ) présentent un couvercle comprenant un piquage de prélèvement permettant de venir prélever le contenu du seau. Pour cela le piquage est relié à une canule s'étendant à l'intérieur de la cuve jusqu'à atteindre le fond du seau. Durant l'étape de préparation du milieu de culture, le piquage de prélèvement est obturé par un bouchon. Une fois le milieu ou le diluant préparé, l'opérateur vient retirer le bouchon et connecte le piquage de prélèvement à une pompe péristaltique de manière à venir aspirer le contenu du seau par l'intermédiaire de la canule.

Cependant, lors de la fermeture du couvercle de l'auto-préparateur, la pression exercée par le mouvement du couvercle sur le contenu du seau peut entrainer une remontée rapide du contenu par la canule. Si l'opérateur n'a pas serré correctement ou a omis d'ajouter le bouchon du piquage de prélèvement ou d'autres bouchons présents sur le couvercle, le contenu du seau se déverse alors à l'extérieur de la cuve ce qui n'est évidemment pas souhaitable. D'autre part, lors de la montée en température et en pression de l'appareil, la vapeur contenue dans la cuve va exercer une pression sur le contenu liquide du seau. Si le bouchon du piquage de prélèvement est mal serré ou absent, le contenu du seau va alors chercher à s'échapper en remontant par la canule et se déverser à l'extérieur de la cuve, le contenu pouvant avoir déjà atteint une température dangereuse pour l'opérateur. D'autre part, une fuite de vapeur par le bouchon de piquage peut également se produire lors de la montée en température et en pression de l'appareil si celui-ci est mal serré.

Ce déversement est d'autant peu souhaitable qu'il entraine la perte partielle voire totale du milieu préparé, la stérilité de celui-ci n'étant plus garantie. De plus l'opération de nettoyage de l'appareil lorsqu'il est recouvert de milieu gélosé est particulièrement fastidieuse.

Ce déversement peut également se produire lors de tests de mise en pression de la cuve à l'aide d'un compresseur. Ce type de test peut notamment être effectué en début du cycle pour vérifier l'étanchéité du ou des bouchons du couvercle obturant la cuve.

Il existe donc un besoin non satisfait de proposer un appareil pressurisé permettant de garantir la sécurité des opérateur et des biens entourant l'appareil pressurisé tout en permettant le prélèvement simple et rapide de la totalité du contenu de l'appareil pressurisé suite à sa préparation.

Un autre objectif de l'invention est de proposer un appareil pressurisé d'entretien simple et rapide.

Pour atteindre ces objectifs, il a été mis au point un appareil pressurisé comprenant :
- une cuve, apte à réceptionner un contenu liquide
- un couvercle, apte à obturer la cuve dans une position de fermeture,
- ledit couvercle comprenant un embout de prélèvement, ledit embout comprenant une canule susceptible d'être en contact avec le contenu lorsque le couvercle est en position de fermeture
- l'embout de prélèvement comprend au moins un orifice obturable permettant, lorsque le couvercle est dans sa position de fermeture et ledit orifice est débouchant d'assurer un équilibre de pression entre l'intérieur de la canule et l'intérieur de la cuve, et lorsque le couvercle est dans sa position de fermeture et ledit orifice est obturé de prélever le contenu via la canule.

Avantageusement, l'embout de prélèvement comprend plusieurs orifices obturables, répartis radialement sur la partie de l'embout s'étendant dans la cuve, préférentiellement à proximité du couvercle.

Avantageusement, l'embout de prélèvement comprend une partie intermédiaire amovible par rapport au couvercle, préférentiellement par vissage.

Avantageusement, ladite partie intermédiaire est apte à coopérer avec un moyen de serrage, ladite partie s'étendant de préférence dans la cuve lorsque le couvercle est en position fermée.

Avantageusement, l'appareil pressurisé comprend un préleveur amovible, ledit préleveur amovible obturant l'orifice obturable quand celui-ci est disposé et maintenu dans l'embout de prélèvement.

Avantageusement, le préleveur amovible est apte à coopérer avec un moyen d'aspiration tel qu'une pompe péristaltique.

Avantageusement, l'appareil pressurisé comprend un bouchon, l'orifice(s) étant débouchant et l'embout de prélèvement étant obturé lorsque le bouchon est disposé et maintenu sur l'embout de prélèvement.

Avantageusement, la canule comprend une extrémité biseauté.

Avantageusement, la canule comprend deux parties connectables, au moins une des parties connectables étant de préférence flexible

Avantageusement, la cuve présente un fond galbé.

Avantageusement, la cuve est apte à réceptionner un seau afin de contenir le contenu liquide, la canule s'étendant dans le seau lorsque le couvercle est en position de fermeture.

L'invention concerne également l'utilisation d'un appareil pressurisé tel que décrit précédemment pour la préparation d'un milieu de culture.

On entend par seau une pièce comprenant une paroi cylindrique ou en tronc de cône et un fond, apte à contenir des constituants tel que des liquides, poudres ou gel, notamment pour la préparation d'un milieu de culture à stériliser. Au sens de la présente invention, un seau est généralement réalisée en acier inoxydable, par emboutissage. Le fond du seau peut présenter un galbe orienté vers l'intérieur du seau de manière à répartir un contenu liquide dans une zone annulaire située à proximité du contact entre le fond du seau et la paroi du seau. Ce galbe à l'avantage de faciliter le prélèvement du liquide en minimisant la surface sur laquelle celui-ci est réparti à mesure que le niveau de liquide à prélever diminue.

On entend par cuve une cavité emménagée dans le bâti d'un appareil pressurisé, susceptible de recevoir un seau. Une cuve peut être composée de trois parties, généralement soudés entre elles, un plateau, une paroi et un fond. Le plateau est une pièce plane dans lequel est ajourée une partie généralement circulaire, correspondant à la partie débouchante de la cuve. La cuve comprend également une paroi cylindrique ou en tronc de cône, en liaison avec la partie débouchante et le fond. La paroi peut être réalisée par une virole découpée puis roulée et soudée au plateau. Le fond est généralement embouti puis soudé à la paroi. Le plateau de la cuve correspond à la partie supérieure du bâti de sorte que la fermeture du couvercle entraine l'obturation de la cuve.

Par couvercle on entend un ensemble mobile par rapport au bâti, susceptible d'obturer la cuve dans une position de fermeture. Afin de garantir la sécurité du personnel au voisinage de l'appareil et la tenue en pression, le couvercle présente également une position de verrouillage garantissant une fermeture hermétique de la cuve et une résistance à la pression jusqu'à un certain seuil, généralement défini selon les réglementations en vigueur dans le pays d'utilisation de l'appareil.

L'invention sera bien comprise et d'autres caractéristiques et avantages de l'invention ressortiront clairement de la description qui en est faite ci-après, à titre indicatif et nullement limitatif, en référence au dessin, dans lequel :
- la figure 1 est une représentation en perspective d'un appareil pressurisé selon l'invention
- la figure 2 est une représentation en vue éclatée du couvercle de l'appareil pressurisé
- la figure 3 est une représentation en coupe de l'appareil pressurisé
- la figure 4 est une représentation en vue éclatée de l'embout de prélèvement de l'appareil pressurisé
- la figure 5 est une représentation en coupe du couvercle, les orifices obturables de l'embout de prélèvement étant débouchant
- la figure 6 est une représentation en coupe du couvercle, les orifices obturables de l'embout de prélèvement étant obturés
- la figure 7 est une représentation en coupe du couvercle, l'embout de prélèvement étant obturé par un bouchon et les orifices obturables étant débouchant

Par simplification, les parties ou éléments d'une forme de réalisation qui se retrouvent de manière identique ou similaire dans une autre forme de réalisation seront identifiés par les mêmes références numériques et ne seront pas à nouveau décrits.

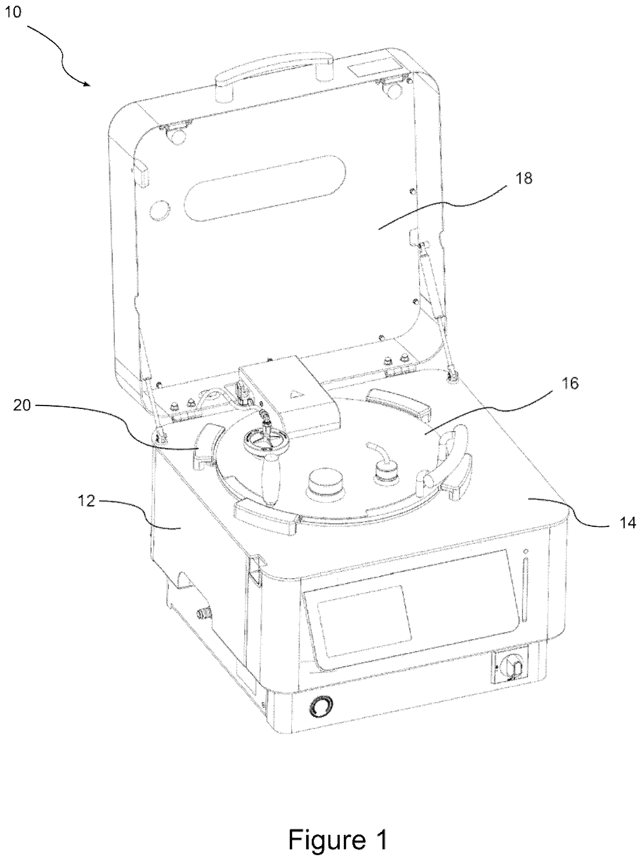

Selon un mode de réalisation de l'invention, la figure 1 représente un appareil pressurisé, également appelé auto-préparateur 10 comprenant un bâti 12 présentant une partie supérieure ou plateau 14 dans lequel est aménagé une cuve. La cuve est présentée obturée par un couvercle 16 pouvant être protégé par un carter amovible 18. La cuve est apte à réceptionner un seau. Le couvercle 16 peut être maintenu dans une position de verrouillage par quatre crabots 20 en forme de L faisant sailli sur le plateau 14.

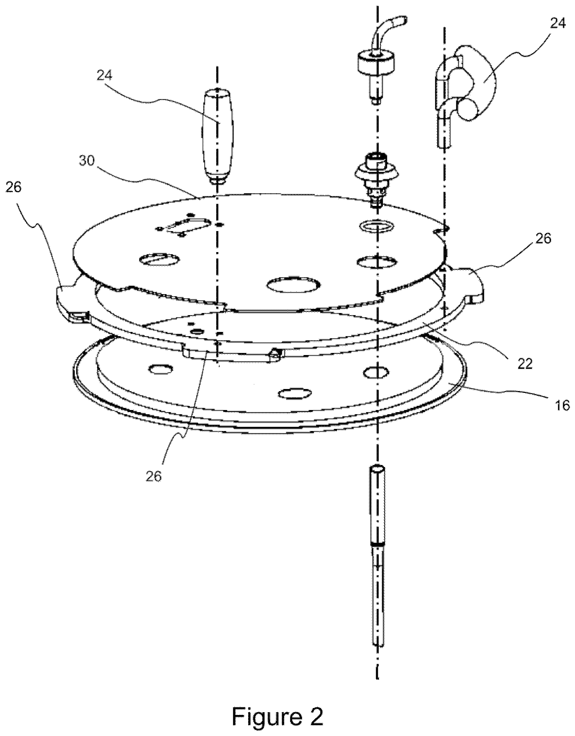

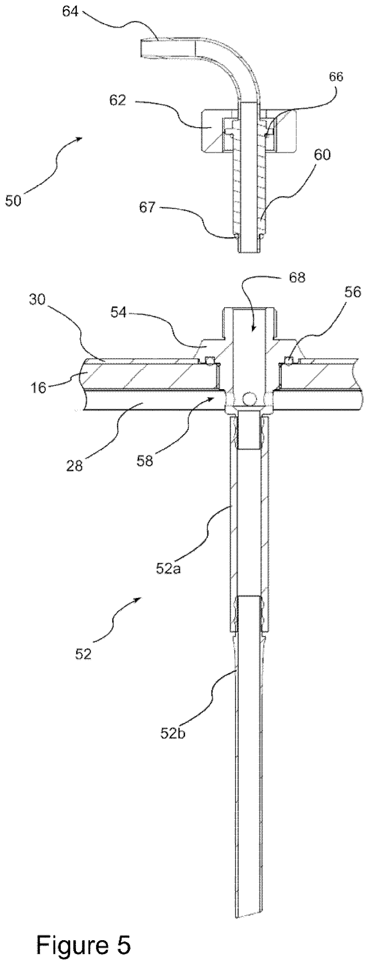

Pour cela et comme représenté sur la figure 2, le couvercle 16 comprend une couronne 22, solidaire et mobile en rotation par rapport au couvercle et apte à se déplacer d'une position libre à une position de verrouillage par l'intermédiaire de deux poignées 24, ladite couronne comprenant quatre ergots de verrouillage 26 s'étendant radialement et coopérant avec les crabots 20 lorsque le couvercle est en position de fermeture et que ladite couronne est en position de verrouillage de manière à pouvoir pressuriser la cuve. Le couvercle présente également un joint d'étanchéité à lèvre 28, visible sur les figures 5 à 7 et un capot 30. Le joint est solidaire du couvercle de manière à assurer l'étanchéité de la cuve lorsque le couvercle est en position de fermeture et la couronne en position de verrouillage.

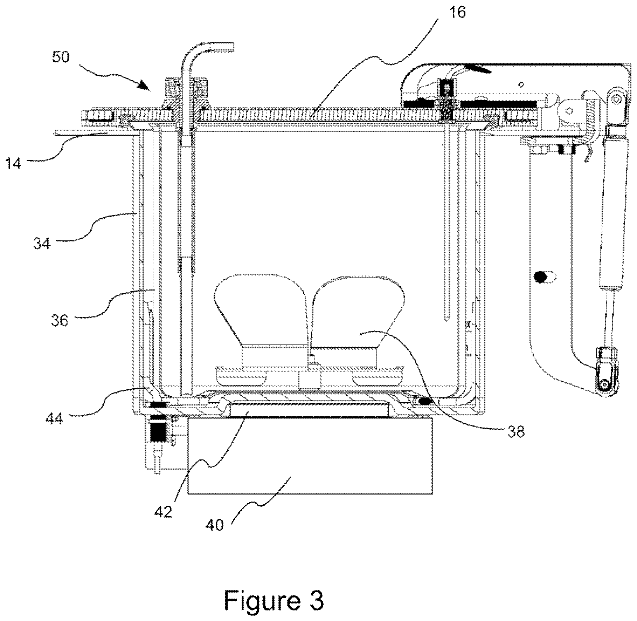

Comme représenté sur la figure 3, la cuve 34 est aménagée dans le plateau 14 et contient un seau 36. Un agitateur magnétique 38 est disposé au fond du seau. L'agitateur magnétique est une pièce en matériau ferromagnétique tel qu'en acier inoxydable, permettant d'homogénéiser la température du contenu du seau en effectuant des rotations cycliques. L'agitateur magnétique permet par exemple d'homogénéiser un milieu de culture en préparation et d'éviter l'apparition de déchets dus par exemple à une surchauffe locale du milieu. Un moteur 40 est fixé sous la cuve et fait tourner un plateau 42 où sont présents des aimants, entrainant ainsi l'agitateur disposé dans le seau, au contact du contenu. Trois résistances chauffantes 44 sont disposées dans la cuve autour du seau et permettent de réguler la température de la cuve et du contenu du seau.

Un embout de prélèvement 50 est vissé dans le couvercle 16.

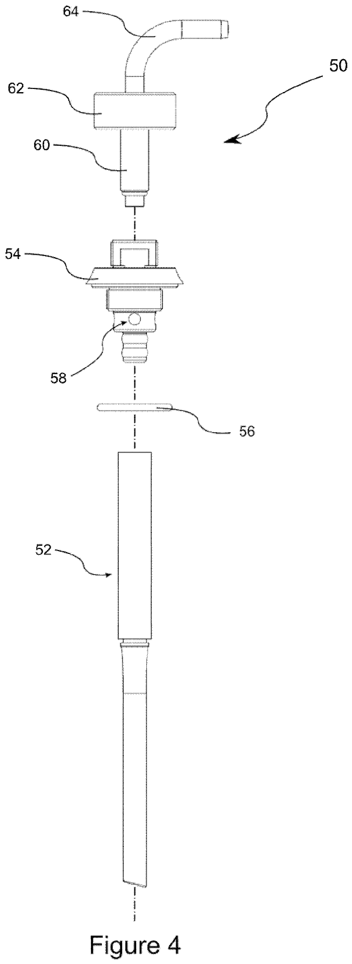

Comme représenté sur les figures 4 et 5, l'embout de prélèvement 50 comprend une canule 52, destinée à s'étendre dans la cuve, plus précisément dans le seau, de manière à pouvoir prélever le contenu du seau par aspiration au travers de la canule. La canule 52 est avantageusement réalisée en deux parties connectables 52a, 52b, préférentiellement emboitées. La partie 52b présente une extrémité biseautée coopérant avec le fond galbé du seau afin de pouvoir prélever une quantité maximale de contenu. La partie 52a est avantageusement réalisée en une matière flexible tel que du silicone afin de faciliter l'ouverture et la fermeture du couvercle de la cuve. La partie 52b est avantageusement réalisée en acier inoxidable ou tout autre matériau permettant de lester suffisamment la canule 52 pour que l'extrémité de celle-ci soit le plus proche possible du fond de la cuve ou du seau quand le couvercle de la cuve est fermé. La canule 52 est elle même emboitée dans une pièce intermédiaire 54 destinée à être vissée sur le couvercle 16 de l'appareil. L'étanchéité du vissage est assurée par un joint torique 56. La pièce intermédiaire 54 est débouchante, permettant ainsi de créer un canal de prélèvement 68 entre l'extérieur de l'appareil et la canule quand celle-ci est emboitée. Le canal de prélèvement 68 s'étend selon un axe longitudinal similaire à celui de la canule. La pièce intermédiaire 54 comprend également quatre orifices obturables 58 reliant la cuve, l'intérieur de la canule et le canal de prélèvement 68 quand ceux-ci sont débouchants. Les quatre orifices obturables 58 sont répartis radialement autour de l'axe du canal de prélèvement 68.

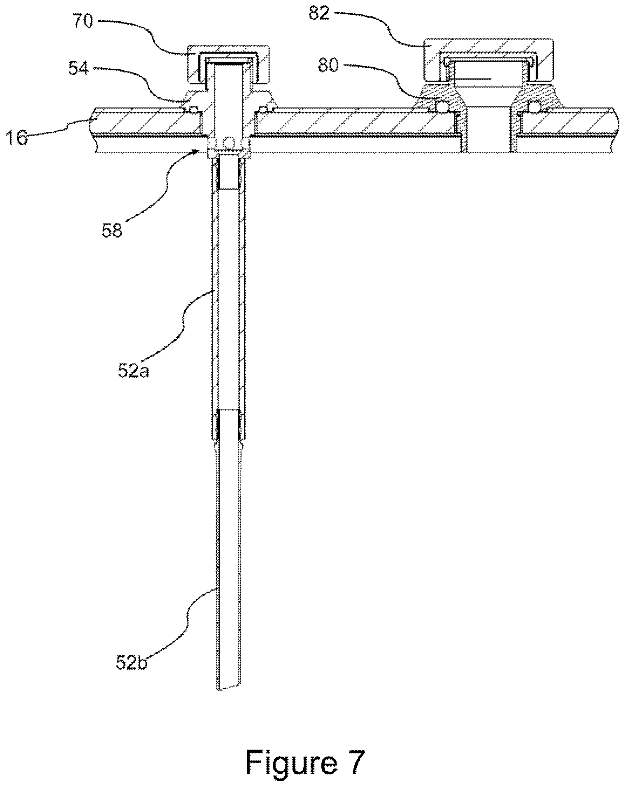

L'embout de prélèvement 50 peut être accouplé à un préleveur amovible 60 pouvant être inséré dans le canal de prélèvement 68 et vissé sur la partie intermédiaire 54 par une bague 62. L'embout de prélèvement 50 peut également être obturé par un bouchon 70, représenté sur la figure 7, vissé sur la partie intermédiaire 54 et obturant ainsi le canal de prélèvement 68 tout en laissant les orifices obturables 58 débouchant afin de permettre la circulation de l'air entre l'intérieur de la canule 52 et la cuve.

Afin de réaliser une étape de préparation de milieu de culture ou de diluant, le canal de prélèvement 68 est obturée par un bouchon 70 vissé sur la partie intermédiaire 54. Dans le cas ou une autre ouverture 80 est présente sur le couvercle, celle-ci est également obturée à l'aide d'un bouchon 82, ici un bouchon vissé, visible sur la figure 7. Lors de cet étape, l'air circule librement entre l'intérieur de la canule est les orifices obturables 58, empêchant ainsi la création d'un différentiel de pression entre ces deux cavités pouvant entrainer la remontée du liquide dans la canule. Afin de pressuriser la cuve, le couvercle comprend un piquage pour une arrivée d'air en provenance d'un compresseur. Cette entrée possède un filtre pour que l'air qui y passe reste également stérile. Lors de la chauffe de la cuve, avant que la température du milieu n'atteigne 100°C, de la vapeur se forme dans la cuve mais celle-ci ne passe pas par ce filtre grâce à un clapet anti-retour. La vapeur ainsi formée reste contenue dans la cuve et circule librement dans la canule, exerçant également une pression sur le contenu liquide de la canule. Le niveau de liquide dans la canule est alors similaire à celui du seau.

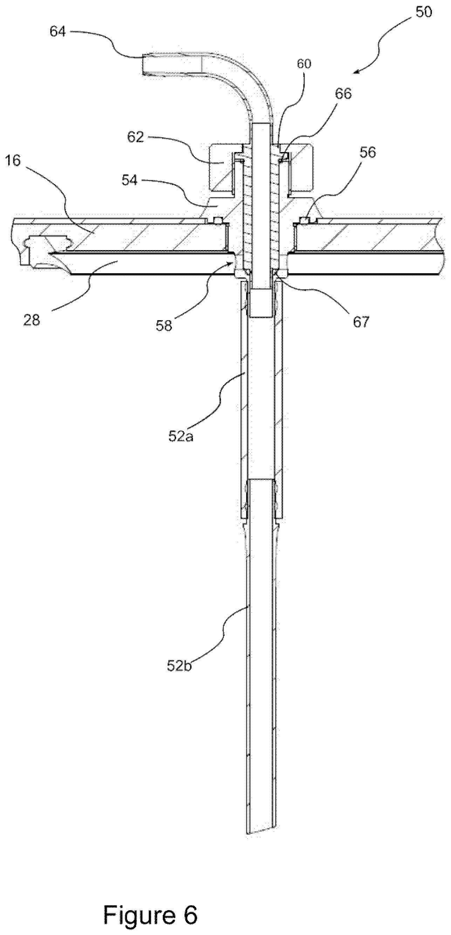

L'étape de préparation du milieu de culture ou diluant se termine par le refroidissement de la cuve. Le volume occupé par la vapeur dans la cuve diminue alors, créant une dépression. De l'air est alors aspiré par le filtre ci-dessus afin de rééquilibrer la pression de la cuve avec la pression atmosphérique. Une fois l'équilibre établi, l'opérateur vient alors visser le préleveur 60 sur la partie intermédiaire 54 en lieu et place du bouchon, tel que représenté sur la figure 6. Le préleveur amovible 60 est préalablement stocké dans un sachet stérile et autoclavé entre chaque cycle de préparation. L'étanchéité du vissage du préleveur une fois vissé est assurée par deux joints toriques 66 et 67. Cette configuration permet de proposer un préleveur amovible 60 simple et compact, pouvant être facilement manipulé et stérilisé entre chaque opération, notamment grâce au fait que la canule reste solidaire du couvercle et n'a pas à être manipulée lors du prélèvement du milieu préparé.

Le préleveur amovible 60 présente une partie cylindrique s'étendant selon un axe longitudinal dont le diamètre extérieur est ajusté de façon serrée avec le diamètre intérieur du canal de prélèvement 68 de la pièce intermédiaire 54. Cet ajustement ainsi que le joint torique 67 disposé entre l'extrémité du préleveur amovible 60 est la pièce intermédiaire 54, permettent d'obturer les orifices obturables 58 quand celui-ci est inséré et vissé sur la pièce intermédiaire. Celui-ci empêche donc l'air de circuler de l'intérieur, la partie non immergée, de la cuve vers l'intérieur de la canule et vers l'extérieur, tant que la cuve contient un liquide à prélever. Le préleveur amovible 60 présente une extrémité 64 pouvant être connectée à un moyen de prélèvement tel qu'une pompe péristaltique ou un distributeur de milieux de culture tel que l'APS ONE, commercialisé par la demanderesse. L'opérateur vient alors connecter un moyen de prélèvement sur l'extrémité 64 afin de pomper le milieu préparé.

Alternativement, le préleveur comprend une partie cylindrique comprenant quatre orifices coopérant avec les orifices obturables de la pièce intermédiaire. Dans une première position du préleveur, les orifices sont disposés en concordance avec les orifices obturables de façon à assurer un équilibre de pression entre la cuve et l'intérieur de la canule. Le préleveur peut alors être fermé par un bouchon, de façon à pouvoir pressuriser la cuve.

Dans une seconde position du préleveur, la partie cylindrique est déplacée par rotation ou translation de manière à obturer les orifices obturables. La préleveur peut alors être connecté à un moyen de prélèvement, de façon à pomper le contenu de la cuve.

## Revendications

1. Appareil pressurisé (10) comprenant :
- une cuve (34), apte à réceptionner un contenu liquide,
- un couvercle (16), apte à obturer la cuve dans une position de fermeture,
- ledit couvercle comprenant un embout de prélèvement (50), ledit embout comprenant :
o une canule (52) susceptible d'être en contact avec le contenu lorsque le couvercle est en position de fermeture,
o et au moins un orifice obturable (58) permettant, lorsque le couvercle est dans sa position de fermeture et ledit orifice est débouchant d'assurer un équilibre de pression entre l'intérieur de la canule et l'intérieur de la cuve, et lorsque le couvercle est dans sa position de fermeture et ledit orifice est obturé de prélever le contenu via la canule,
**caractérisé en ce qu'**il comprend un préleveur amovible (60), ledit préleveur amovible (60) obturant l'orifice obturable (58) quand celui-ci est disposé et maintenu dans l'embout de prélèvement (50).

2. Appareil pressurisé selon la revendication 1, **caractérisé en ce que** le préleveur amovible (60) est apte à coopérer avec un moyen d'aspiration tel qu'une pompe péristaltique.

3. Appareil pressurisé selon la revendication 1 ou 2, **caractérisé en ce que** l'embout de prélèvement (50) comprend plusieurs orifices obturables (58), répartis radialement sur la partie de l'embout s'étendant dans la cuve, préférentiellement à proximité du couvercle.

4. Appareil pressurisé selon l'une des revendications précédentes, **caractérisé en ce que** l'embout de prélèvement (50) comprend une partie intermédiaire amovible (54) par rapport au couvercle, préférentiellement par vissage.

5. Appareil pressurisé selon la revendication précédente, **caractérisé en ce que** la partie intermédiaire (54) est apte à coopérer avec un moyen de serrage, ladite partie s'étendant de préférence dans la cuve lorsque le couvercle est en position de fermeture.

6. Appareil pressurisé selon l'une des revendications précédentes comprenant un bouchon (70) coopérant avec l'embout de prélèvement (50), **caractérisé en ce que** l'orifice(s) est débouchant et l'embout de prélèvement est obturé lorsque le bouchon (70) est disposé et maintenu sur l'embout de prélèvement (50).

7. Appareil pressurisé selon l'une des revendications précédentes, **caractérisé en ce que** la canule (52) comprend une extrémité biseauté.

8. Appareil pressurisé selon l'une des revendications précédentes, **caractérisé en ce que** la canule (52) est réalisé en deux parties connectables (52a, 52b), au moins une des parties (52a, 52b) étant de préférence flexible.

9. Appareil pressurisé selon l'une des revendications précédentes, **caractérisé en ce que** la cuve (34) présente un fond galbé.

10. Appareil pressurisé selon l'une des revendications précédentes, **caractérisé en ce que** la cuve (34) est apte à réceptionner un seau (36) afin de contenir le contenu liquide, la canule (52) s'étendant dans le seau lorsque le couvercle est en position de fermeture.

11. Utilisation d'un appareil pressurisé (10) selon l'une des revendications précédentes pour la préparation d'un milieu de culture.

## Patentansprüche

1. Unter Druck stehendes Gerät (10), welches umfasst:
- einen Tank (34), der geeignet ist, einen flüssigen Inhalt aufzunehmen,
- einen Deckel (16), der geeignet ist, den Tank in einer Schließposition zu verschließen,
- wobei der Deckel einen Entnahmestutzen (50) umfasst, wobei dieser Stutzen umfasst:
o ein Röhrchen (52), das dazu eingerichtet ist, in Kontakt mit dem Inhalt zu stehen, wenn sich der Deckel in der Schließposition befindet,
o und mindestens eine verschließbare Öffnung (58), welche ermöglicht, wenn sich der Deckel in seiner Schließposition befindet und die Öffnung offen ist, ein Druckgleichgewicht zwischen dem Inneren des Röhrchens und dem Inneren des Tanks sicherzustellen, und wenn sich der Deckel in seiner Schließposition befindet und die Öffnung verschlossen ist, den Inhalt über das Röhrchen zu entnehmen,
**dadurch gekennzeichnet, dass** es eine lösbare Entnahmevorrichtung (60) umfasst, wobei die lösbare Entnahmevorrichtung (60) die verschließbare Öffnung (58) verschließt, wenn sie im Entnahmestutzen (50) angeordnet ist und gehalten wird.

2. Unter Druck stehendes Gerät nach Anspruch 1, **dadurch gekennzeichnet, dass** die lösbare Entnahmevorrichtung (60) geeignet ist, mit einem Ansaugmittel wie etwa einer peristaltischen Pumpe zusammenzuwirken.

3. Unter Druck stehendes Gerät nach Anspruch 1 oder 2, **dadurch gekennzeichnet, dass** der Entnahmestutzen (50) mehrere verschließbare Öffnungen (58) umfasst, die radial auf dem Teil des Stutzens verteilt sind, der sich im Tank erstreckt, vorzugsweise in der Nähe des Deckels.

4. Unter Druck stehendes Gerät nach einem der vorhergehenden Ansprüche, **dadurch gekennzeichnet, dass** der Entnahmestutzen (50) einen Zwischenteil (54) umfasst, der bezüglich des Deckels lösbar ist, vorzugsweise durch eine Schraubverbindung.

5. Unter Druck stehendes Gerät nach dem vorhergehenden Anspruch, **dadurch gekennzeichnet, dass** der Zwischenteil (54) geeignet ist, mit einem Spannmittel zusammenzuwirken, wobei sich dieser Teil vorzugsweise im Tank erstreckt, wenn sich der Deckel in der Schließposition befindet.

6. Unter Druck stehendes Gerät nach einem der vorhergehenden Ansprüche, welches ein Verschlussstück (70) umfasst, das mit dem Entnahmestutzen (50) zusammenwirkt, **dadurch gekennzeichnet, dass** die Öffnung(en) offen ist (sind) und der Entnahmestutzen verschlossen ist, wenn das Verschlussstück (70) auf dem Entnahmestutzen (50) angeordnet ist und gehalten wird.

7. Unter Druck stehendes Gerät nach einem der vorhergehenden Ansprüche, **dadurch gekennzeichnet, dass** das Röhrchen (52) ein abgeschrägtes Ende umfasst.

8. Unter Druck stehendes Gerät nach einem der vorhergehenden Ansprüche, **dadurch gekennzeichnet, dass** das Röhrchen (52) aus zwei verbindbaren Teilen (52a, 52b) hergestellt ist, wobei mindestens einer der Teile (52a, 52b) vorzugsweise biegsam ist.

9. Unter Druck stehendes Gerät nach einem der vorhergehenden Ansprüche, **dadurch gekennzeichnet, dass** der Tank (34) einen gewölbten Boden aufweist.

10. Unter Druck stehendes Gerät nach einem der vorhergehenden Ansprüche, **dadurch gekennzeichnet, dass** der Tank (34) geeignet ist, einen Eimer (36) aufzunehmen, um den flüssigen Inhalt zu fassen, wobei sich das Röhrchen (52) in dem Eimer erstreckt, wenn sich der Deckel in der Schließposition befindet.

11. Verwendung eines unter Druck stehenden Gerätes (10) nach einem der vorhergehenden Ansprüche für die Herstellung eines Kulturmediums.

## Claims

1. Pressurized appliance (10) comprising:
- a tank (34), suitable for receiving liquid contents,
- a cover (16), suitable for sealing the tank in a closed position,
- said cover comprising a sampling tip (50), said tip comprising:
o a cannula (52) capable of being in contact with the contents when the cover is in the closed position,
o and at least one sealable orifice (58) making it possible, when the cover is in its closed position and said orifice is a through-orifice, to ensure a pressure equilibrium between the inside of the cannula and the inside of the tank, and when the cover is in its closed position and said orifice is sealed, to sample the contents via the cannula,
**characterized in that** it comprises a removable sampler (60), said removable sampler (60) sealing the sealable orifice (58) when the latter is positioned and held in the sampling tip (50).

2. Pressurized appliance according to Claim 1, **characterized in that** the removable sampler (60) is suitable for cooperating with a suction means such as a peristaltic pump.

3. Pressurized appliance according to Claim 1 or 2, **characterized in that** the sampling tip (50) comprises several sealable orifices (58), distributed radially over the part of the tip extending into the tank, preferentially close to the cover.

4. Pressurized appliance according to one of the preceding claims, **characterized in that** the sampling tip (50) comprises an intermediate part (54) that is removable relative to the cover, preferentially by screwing.

5. Pressurized appliance according to the preceding claim, **characterized in that** the intermediate part (54) is suitable for cooperating with a tightening means, said part extending preferably into the tank when the cover is in the closed position.

6. Pressurized appliance according to one of the preceding claims, comprising a cap (70) that cooperates with the sampling tip (50), **characterized in that** the orifice(s) is (are) through-orifice(s) and the sampling tip is sealed when the cap (70) is positioned and held on the sampling tip (50).

7. Pressurized appliance according to one of the preceding claims, **characterized in that** the cannula (52) comprises a bevelled end.

8. Pressurized appliance according to one of the preceding claims, **characterized in that** the cannula (52) is made of two connectable parts (52a, 52b), at least one of the parts (52a, 52b) preferably being flexible.

9. Pressurized appliance according to one of the preceding claims, **characterized in that** the tank (34) has a curved bottom.

10. Pressurized appliance according to one of the preceding claims, **characterized in that** the tank (34) is suitable for receiving a bucket (36) in order to contain the liquid contents, the cannula (52) extending into the bucket when the cover is in the closed position.

11. Use of a pressurized appliance (10) according to one of the preceding claims for the preparation of a culture medium.
